Europäisches Patentamt

(19) **European Patent Office**

Office européen des brevets

(11) Numéro de publication: **0 237 372**
**B1**

(12) **FASCICULE DE BREVET EUROPEEN**

(45) Date de publication du fascicule du brevet: **18.04.90**

(51) Int. Cl.⁵: **C 10 L 1/06**

(21) Numéro de dépôt: **87400220.7**

(22) Date de dépôt: **30.01.87**

(54) Procédé d'obtention de méthyltertiobutyléther de supercarburant et de combustible pour carburéacteur à partir des butanes et/ou des coupes C4 d'un craquage ou d'un reformage catalytique.

(30) Priorité: **13.02.86 FR 8602063**

(43) Date de publication de la demande:
**16.09.87 Bulletin 87/38**

(45) Mention de la délivrance du brevet:
**18.04.90 Bulletin 90/16**

(84) Etats contractants désignés:
**AT BE DE FR GB IT NL**

(56) Documents cités:
**EP-A-0 068 981**
**US-A-4 528 411**
**US-A-4 542 247**

(73) Titulaire: **INSTITUT FRANCAIS DU PETROLE**
**4, Avenue de Bois-Préau**
**F-92502 Rueil-Malmaison (FR)**

(72) Inventeur: **Juguin, Bernard**
**46, avenue du Stade**
**F-92500 Rueil Malmaison (FR)**
Inventeur: **Mabilon, Gil**
**36, rue de Tourville**
**F-78100 Saint Germain en Laye (FR)**
Inventeur: **Martino, Germain**
**Bâtiment Condé 80, avenue F. Lefèbvre**
**F-78300 Poissy (FR)**

Courier Press, Leamington Spa, England.

**Description**

La présente invention concerne un procédé d'obtention de méthyltertiobutyléther, du supercarburant, et de combustible pour carburéacteur, procédé rendu possible par une combinaison judicieuse de plusieurs étapes. La charge initiale est en général une coupe butanes en provenance par exemple d'une unité de reformage catalytique, ou d'une unité de craquage catalytique.

Le procédé selon l'invention est adapté pour les raffineurs et/ou les pétrochimistes possédant une unité de reformage catalytique et/ou une unité de craquage catalytique, et permet de mieux valoriser les coupes butanes qui, le plus souvent sont excédentaires.

Le procédé selon l'invention permet en particulier d'obtenir:

1) du méthyltertiobutyléther

2) du supercarburant de bonne qualité

3) du combustible pour carburéacteur également de bonne qualité.

Le procédé selon l'invention est conçu de façon à ce que:

a) la charge fraîche, constituée en majeure partie d'une coupe $C_4$ paraffinique, préalablement séchée, désulfurée et désazotée, et qui généralement, à ce stade renferme en majeure partie de l'isobutane et du n-butane, est en premier lieu envoyée dans une zone de déshydrogénation où l'isobutane et le n-butane sont au moins en partie transformés en isobutène, butène-1, butènes-2 et butadiène-1,3.

b) L'effluent soutiré de la zone de déshydrogénation de l'étape a) est ensuite refroidi par échange avec de la charge fraîche, puis envoyé dans une première colonne de fractionnement, on recueille en tête un mélange d'hydrogène de méthane et d'éthane riche en hydrogène, la majeure partie de ce mélange est recyclée en amont du réacteur de déshydrogénation afin de contribuer à maintenir la stabilité du catalyseur de déshydrogénation, l'autre partie pouvant être utilisée à l'intérieur de la raffinerie, par exemple dans des unités d'hydrotraitement.

On recueille un premier produit en fond de cette première colonne que l'on envoit dans une deuxième colonne de fractionnement où l'on recueille en tête un second produit constitué principalement de propane, qui sera utilisé comme combustible à l'intérieur de la raffinerie et en fond un deuxième produit renfermant notamment du n-butane, de l'isobutane, des butènes-2, de l'isobutène et du butadiène-1,3.

c) Le second produit soutiré en fond de la seconde colonne est envoyé dans un réacteur d'éthérification ou la majeure partie de l'isobutène qu'il contient va réagir avec du méthanol en excès pour donner du méthyltertiobutyléther ou MTBE.

d) L'effluent soutiré de la zone d'éthérification de l'étape c) est soumis à une série de fractionnement (au moins deux fractionnements):

— dans la première colonne à distiller traversée par cet effluent on recueille en fond le méthanol en excès qui est recyclé au moins en majeure partie, en amont du réacteur d'éthérification avec le méthanol frais.

— le mélange soutiré en tête de cette première colonne de fractionnement traversé par cet effluent est envoyé dans au moins une autre colonne et l'on recueille en fond de la dernière colonne le méthyltertiobutyléther ou MTBE sensiblement pur.

— le mélange recueilli en tête de cette dernière colonne qui à ce stade du procédé contient de l'isobutane, du n-butane, du butène 1, des butènes-2, du butadiène-1,3 et des traces de méthanol et de MTBE est envoyé dans une unité de lavage à l'eau (eau acidifié de pH environ 4 par exemple), afin d'éliminer ces traces de méthanol et de MTBE qui sont des inhibiteurs pour le catalyseur d'oligomérisation qui constitue la troisième étape du procédé.

— après lavage à l'eau le mélange est alors séché afin d'éliminer sensiblement la totalité de l'eau qu'il contient, cette dernière étant également un inhibiteur pour le catalyseur d'oligomérisation.

e) L'effluent issu du sécheur est ensuite envoyé dans une zone d'oligomérisation, où les n-butènes, les butènes-2 et le butadiène-1,3 sont presqu'entièrement transformés en partie en supercarburant et en partie en combustible pour carburéacteur.

f) L'effluent soutiré de la zone d'oligomérisation de l'étape e), est soumis à une série de fractionnements: (au moins deux zones de fractionnement)

— dans une première colonne à distiller, on recueille en tête un mélange renfermant essentiellement de l'isobutane et du n-butane, ce mélange étant de préférence entièrement recyclé en amont de la section de deshydrogénation où il est mélangé avec la charge fraiche, et avec une grande partie du mélange riche en hydrogène issu de la tête de la première colonne de fractionnement.

— le mélange soutiré en fond de cette première colonne est envoyé dans au moins une autre colonne et l'on soutire en tête de la dernière colonne une coupe essence, et en fond und coupe type base pour carburéacteur qui pourra éventuellement subir une hydrogénation partielle ou totale.

Dans la zone dite de deshydrogénation, les conditions opératoires sont telles que l'isobutane et le n-butane sont transformés en isobutène et n-butènes, dans des conditions de transformation proches de l'équilibre thermodynamique.

Cette réaction de déshydrogénation peut être effectuée en *phase vapeur*, en présence d'un catalyseur à base de platine et de divers promoteurs déposes sur une alumine dont la surface spécifique est comprise entre 60 et 400 $m^2/g$, à une température d'environ 500 à 650°C (de préférence entre 550 et 600°C), sous une pression de 0,01 à 1 MPa (de préférence entre 0,03 et 0,2 MPa), avec un débit d'hydrocarbures liquides

2

(vitesse spatiale) d'environ 1 à 10 volumes par volume de catalyseur et par heure; afin d'assurer la stabilité du catalyseur, le rapport molaire hydrogène/hydrocarbures à l'entrée du réacteur de déshydrogénation sera compris entre 1/1 et 10/1.

La réaction dite d'éthérification entre l'isobutène contenu dans le produit issu de la zone de déshydrogénation et le méthanol, peut être effectuée en présence d'un catalyseur acide, par exemple l'acide sulfurique, l'acide fluorhydrique, le chlorure d'aluminium et le fluorure de bore. On peut préférer toutefois les matières carbonées contenant des groupes —$SO_3H$, par exemple des charbons sulfonés (par exemple Nalcite X ou AX, ZeO-Karb H), des résines phénol-formaldéhyde sulfonées (par exemple Amberlite IR—1 ou IR—100, Nalcite MX), des polymères coumarone-indène sulfonés ou, de préférence, des résines polystyrène-divinylbenzène sulfonées, par exemple Dowex 50, Nalcite HCR et Amberlyst 15.

Quand on opère en continu, le volume de charge traitée par volume de catalyseur et par heure est habituellement de 0,5 à 20. On opère généralement entre 20 et 150°C, de préférence 40—100°C, avec 1 à 10 moles de méthanol par mole d'isobutène, un excès d'alcool favorisant la réaction, et sous une pression comprise entre 0,1 et 5 MPa. La réaction d'éthérification est bien connu et décrite par exemple dans US—A—2480940.

La réaction dite d'oligomérisation est effectuée en phase liquide ou en phase supercritique, en présence d'un catalyseur acide disposé par exemple sous forme d'un lit fixe, à une température d'environ 80 à 200°C (de préférence 130 à 170°C), sous une pression de 2 à 10 MPa (de préférence entre 3 et 6 MPa), avec un débit d'hydrocarbures liquides (vitesse spatiale) d'environ 0,1 à 2 volumes par volume de catalyseur et par heure.

Les catalyseurs utilisés pour cette réaction d'oligomérisation sont choisis parmi les silice-alumines, les silice-magnésies, les bores-alumines, l'acide phosphorique sur kieselgurh ou sur silice ou sur quartz, et parmi des catalyseurs du type "acide phosphorique solide", c'est à dire un catalyseur constitué d'un matière siliceuse à grand pouvoir absorbant imprégnée d'une proportion élevée d'acide phosphorique, des mélanges de gels d'alumine et de thorine co-précipités ou non, avec éventuellement des additions de chrome, d'oxyde de zinc, ou d'un métal équivalent. On peut encore choisir des catalyseurs obtenus par traitement d'alumine de transition au moyen d'un dérivé acide du fluor, avec éventuellement addition d'un ester silicique. De préférence, on utilise une silice-alumine dont la teneur en silice est comprise entre 60 et 95% en poids, renfermant éventuellement entre 0,1 et 5% en poids d'oxyde de chrome et/ou d'oxyde de zinc.

La réaction éventuelle d'hydrogenation partielle ou totale de la base type combustible pour carburéacteur, est effectuée en présence d'un catalyseur disposé par exemple sous forme d'un lit fixe, le dit catalyseur étant constitué par un mélange hydrogénant déposé sur un support sensiblement neutre, par exemple le catalyseur LD 265 commercialisé par la Société PROCATALYSE (palladium déposé sur alumine). La température de réaction est généralement comprise entre 150 et 400°C, sous une pression de 2 à 10 MPa (de préférence la température est d'environ 220 à 300°C et la pression.4 à 6 MPa), avec un débit d'hydrocarbures liquides (vitesse spatiale) d'environ 1 à 5 volumes par volume de catalyseur et par heure, le rapport molaire hydrogène/hydrocarbures à l'entrée du réacteur étant compris entre 2 et 10.

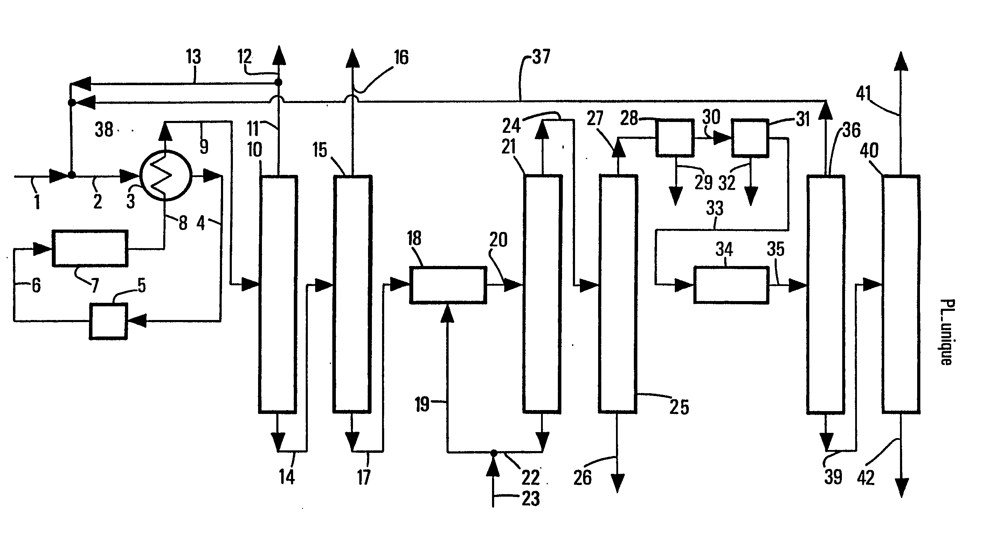

La figure unique illustre l'invention.

La charge fraîche de butanes et/ou de coupe $C_4$ est introduite dans l'unité par la conduite 1, puis elle est mélangée avec l'hydrogène de recyclage en provenance de la tête de la première colonne de fractionnement (10) par les conduites 13 et 38, et avec les butanes non transformés en provenance de la tête de la cinquième colonne de fractionnement (36) par la conduite 37. Le mélange résultant est introduit par la conduite 2 dans un échangeur charge-effluent (3) où il est préchauffé, et à la sortie par la conduite 4, le mélange préchauffé passe à travers un four (5) où il est porté à la température nécessaire à la réaction de déshydrogénation, le dit mélange étant ensuite introduit par la conduite 6 dans la section de déshydrogénation (7). A la sortie de la section de déshydrogénation, l'effluent est introduit par la conduite 8 dans l'échangeur de chaleur charge-effluent (3) où il est refroidi, puis introduit par la conduite 9 dans la première colonne de fractionnement (10). En tête de la colonne (10) par la conduite 11, on recueille un mélange hydrogène méthane éthane très riche en hydrogène; la majeure partie de ce mélange est recyclée par la conduite 13 vers la section de déshydrogénation, l'autre partie est évacuée par la conduite 12, par exemple vers une section d'hydrotraitement située dans la raffinerie. Le produit soutiré en fond de la colonne (10) est envoyé par la conduite 14 vers une seconde colonne de fractionnement (15); en tête de cette colonne (15) on recueille un mélange constitué principalement de propane, d'isobutane, qui est évacué par la conduite 16 vers un autre secteur de la raffinerie, où il pourra être utilisé comme combustible dans un four; par exemple il peut être utilisé comme un appoint de combustible dans le four (5) de la section de déshydrogénation. Le produit soutiré en fond de la colonne (15) est tout d'abord compressé (dispositif non représenté sur la figure), puis envoyé par la conduite 17 vers la section d'éthérification (18). Dans cette section d'éthérification (18) le méthanol frais arrivant par la conduite 23, est alors mélangé avec le méthanol en excès recyclé par la conduite 22 depuis le fond de la troisième colonne de fractionnement (21), le mélange résultant étant introduit après préchauffage ou non, dans la section d'éthérification (18) par la conduite 19. A la sortie de la zone d'éthérification (18), l'effluent est envoyé par la conduite 20, vers une colonne à distiller (21) doù l'on recueille en fond le méthanol en excès qui est recyclé au moins en majeure partie par les conduites 22 et 19 vers la zone d'éthérification. En tête de cette colonne (21), on soutire un mélange constitué d'isobutane, de n-butane, des n-butènes, du butadiène, du MTBE et d'un peu de méthanol, le dit mélange étant envoyé dans une nouvelle colonne à distiller (25) par la conduite 24. En fond de cette colonne (25) on recueille du MTBE sensiblement pur par la conduite 26. En tête de cette colonne (25), on recueille un mélange constitué d'isobutane, de n-butane, de n-butènes, de butadiène-1,3 et d'un peu de méthanol et de MTBE qui est envoyé par la conduite 27 vers une unité de lavage à l'eau acidifiée (28) (pH 4 environ de préférence), ou l'on élimine, par la conduite 29, le méthanol et le MTBE entrainé avec les hydrocarbures en $C_4$. A la sortie de cette unité de lavage à l'eau, la coupe $C_4$ saturée d'eau est envoyé par la conduite 30 vers une unité de séchage (31) où elle est débarassée de l'eau en excès par la conduite 32. L'effluent $C_4$ du sécheur (31) est envoyé, après compression et préchauffage (non représentés sur la figure) vers l'unité d'oligomérisation (34) par la conduite 33. A la sortie de la section d'oligomérisation, l'effluent est détendu, (dispositif non représenté sur la figure) puis introduit par la conduite 35 dans une colonne de fractionnement (36). En tête de cette colonne (36) on recueille un mélange constitué d'isobutane et de n-butane qui est de préférence entièrement recyclé par la conduite 37 vers la section de déshydrogénation. Le produit soutiré en fond de la colonne (36) est envoyé, par la conduite 39, vers une dernière colonne à distiller (40), d'où l'on sépare en tête, par la conduite 41, une coupe essence qui est envoyée par la conduite 41 vers un pool supercarburant. Le produit soutiré en fond de cette colonne (40) constitue une bonne base pour carburéacteur, il peut être soit directement envoyé vers un pool carburéacteur par la conduite 42, soit hydrogéné totalement ou partiellement afin d'en améliorer ses qualités; dans ce cas, une partie au moins de l'hydrogène en provenance de la tête de la colonne (10) par la conduite 12 peut être avantageusement utilisé à cet effet.

Exemple 1

Le bilan matière dans les diverses sections de l'unité globale est présenté sur le tableau I. On traite un mélange de coupe $C_4$ de reformage et de méthanol (en poids: coupe $C_4$ 80,5%, méthanol 19,5%).

La coupe $C_4$ de reformage avait la composition pondérale suivante:

| isobutane | : | 44,94 |
|-----------|---|-------|
| n-butane | : | 54,95 |
| butène-1 | : | 0,11 |
| | | 100 |

Il ressort du tableau I, que si on avait traité 100 kg d'un mélange de coupe $C_4$ et de méthanol, à l'issue du procédé, on obtient les productions nettes suivantes pour 100 kg de mélange coupe $C_4$-méthanol:

| MTBE (ligne 26 de la figure) | : | 52 kg |
|------------------------------|---|-------|
| Supercarburant (ligne 41 de la figure) | : | 27,4 kg |
| Base pour carburéacteur (ligne 42 de la figure) | : | 9,8 kg |
| | | 89,2 kg |

Pour l'étape de déshydrogénation, on utilise un catalyseur à base d'alumine gamma (220 m²/g de surface spécifique, volume poreux 58 cm³/100 g) renfermant en poids 0,3% de platine et 0,08% d'étain). On opère à 580°C sous environ 0,1 MPa (environ 1 bar), avec une vitesse spatiale de 4 $h^{-1}$ et un rapport $H_2$/HC de 3.

Pour l'étape d'oligomérisation, on utilise un catalyseur à base de silice-alumine à 80% poids de silice. On opère à 150°C, sous 55 bars environ (5,5 MPa), avec une vitesse spatiale de 0,5 $h^{-1}$.

Conditions expérimentales dans la zone d'éthérification 18:
T: 50—90°C
P: 12—20 bars

Le catalyseur utilisé est une résine échangeuse d'ions sulfoniques à base de polystyrène réticulé par du divinylbenzène (Amberlyst 15).

Le supercarburant issu de la tête de la colonne (40) par la conduite 41 présentait les caractéristiques suivantes:

— densité à 20°C    :    0,710

— distillation ASTM

Point initial °C    :    39°

10% vol    :    123°

30% vol    :    137°

50% vol    :    152°

70% vol    :    174°

90% vol    :    198°

Point final °C    :    218°

— indice d'octane

RON Clear    :    95

La base pour carburéacteur issue du fond de la colonne (40) par la conduite 42 présentait les caractéristiques suivantes:

— point de fumée    :    22 mm

— point de cristillisation    :    < −65°C.

Après hydrogénation à 80% à l'aide d'hydrogène en provenance de la conduite 12, ces caractéristiques étaient devenues les suivantes:

— point de fumée    :    34 mm

— point de cristallisation    :    < −65°C.

Les conditions opératoires d'hydrogénation étaient les suivantes: catalyseur à base d'alumine de surface spécifique 70 m$^2$/g renfermant 0,3% de palladium en poids. On opère à 270°C sous 50 bars avec une vitesse spatiale égale à 2 h$^{-1}$.

Exemple 2

Le bilan matière dans les diverses sections de l'unité globale est présenté sur le tableau II. On traite un mélange de coupe $C_4$ issue d'une unité de "Fluid Catalytic Cracking" et de méthanol (en poids: coupe $C_4$: 78,3%, méthanol 21,7%).

La coupe $C_4$ de F.C.C. avait la composition pondérale suivante:

isobutane  :    33,80

n-butane  :    11,09

butène-1  :    14,95

isobutène  :    12,63

Σbutènes-2  :    27,53
_____

100

Il ressort du tableau II, que si on avait traité 100 kg d'un mélange de coupe $C_4$ et de méthanol, à l'issue du procédé, on obtient les productions nettes suivantes pour 100 kg de mélange coupe $C_4$-méthanol

EP 0 237 372 B1

| MTBE (ligne 26 de la figure) | : | 57,7 kg |
|---|---|---|
| Supercarburant ligne 41 de la figure) | : | 22,7 kg |
| Base pour carburéacteur (ligne 42 de la figure) | : | 8,1 kg |
| | | 88,5 kg |

Le supercarburant issu de la tête de la colonne (40) par la conduite 41 présentait les caractéristiques suivantes:

| — densité à 20°C | : | 0,710 |
|---|---|---|
| — distillation ASTM | | |
| Point initial °C | : | 47° |
| 10% vol | : | 124° |
| 30% vol | : | 137° |
| 50% vol | : | 153° |
| 70% vol | : | 175° |
| 90% vol | : | 198- |
| Point final °C | : | 219° |
| — indice d'octane | | |
| RON clear | : | 95 |

La base pour carburéacteur issue du fond de la colonne (40) par la conduite 42 présentait les caractéristiques suivantes:

| — point de fumée | : | 22 mm |
|---|---|---|
| — point de cristallisation | : | <−65°C. |

Après hydrogénation à 80%, à l'aide de l'hydrogène en provenance de la conduite 12, ces caractéristiques étaient devenues les suivantes:

| — point de fumée | : | 34 mm |
|---|---|---|
| — point de cristallisation | : | <−65°C. |

Les conditions opératoires de déshydrogénation, éthérification, oligomérisation et hydrogénation sont les mêmes que pour l'exemple 1.

6

TABLEAU I

| Numéros des conduites de la figure | 1 | 6 | 9 | 11 | 12 | 13 | 16 | 17 | 19 | 20 | 22 | 23 | 26 | 27 | 29 | 30 | 32 | 33 | 35 | 37 | 38 | 41 | 42 |
|---|---|---|---|---|---|---|---|---|---|---|---|---|---|---|---|---|---|---|---|---|---|---|---|
| Hydrogène | — | 9,40 | 10,35 | 10,35 | 0,95 | 9,40 | — | — | — | — | — | — | — | — | — | — | — |  |  | — | 9,40 | — | — |
| Méthane | — | 0,74 | 0,82 | 0,82 | 0,08 | 0,74 | — | — | — | — | — | — | — | — | — | — | — |  |  | — | 0,74 | — | — |
| Ethane | — | 0,45 | 0,54 | 0,50 | 0,05 | 0,45 | 0,04 | — | — | — | — | — | — | — | — | — | — |  |  | — | 0,45 | — | — |
| Propane | — | 0,08 | 1,37 | 0,09 | 0,01 | 0,08 | 1,28 | — | — | — | — | — | — | — | — | — | — |  |  | — | 0,08 | — | — |
| Propène | — | — | 0,96 | — | — | — | 0,96 | — | — | — | — | — | — | — | — | — | — |  |  | — | — | — | — |
| Isobutane | 15,81 | 40,12 | 24,86 | — | — | — | 0,69 | 24,17 | — | 24,17 | — | — | — | 24,17 | — | 24,17 | — | 24,17 | 24,31 | 24,31 | 24,31 | — | — |
| n-butane | 17,82 | 49,03 | 32,25 | — | — | — | — | 32,25 | — | 32,25 | — | — | — | 32,25 | — | 32,25 | — | 32,25 | 32,25 | 31,11 | 31,11 | 1,14 | — |
| Butène-1 | 0,11 | 0,18 | 5,06 | — | — | — | — | 5,06 | — | 5,06 | — | — | — | 5,06 | — | 5,06 | — | 5,06 | 0,09 | 0,07 | 0,07 | 0,02 | — |
| Isobutène | — | — | 14,24 | — | — | — | — | 14,24 | — | 0,10 | — | — | — | 0,10 | — | 0,10 | — | 0,10 | — | — | — | — | — |
| ≤ butènes-2 | — | — | 9,40 | — | — | — | — | 9,40 | — | 9,40 | — | — | — | 9,40 | — | 9,40 | — | 9,40 | 0,77 | — | — | 0,77 | — |
| Butadiène-1,3 | — | — | 0,15 | — | — | — | — | 0,15 | — | 0,15 | — | — | — | 0,15 | — | 0,15 | — | 0,15 | — | — | — | — | — |
| Méthanol | — | — | — | — | — | — | — | — | 10,50 | 2,42 | 2,30 | 8,20 | — | 0,12 | 0,12 | — | — | — | — | — | — | — | — |
| MTBE | — | — | — | — | — | — | — | — | 0,22 | 22,22 | 0,22 | — | 22,07 | 0,15 | 0,15 | — | — | — | — | — | — | — | — |
| Eau | — | — | — | — | — | — | — | — | — | — | — | — | — | — | q— | 0,04 | 0,04 | — | — | — | — | — | — |
| Supercarburant | — | — | — | — | — | — | — | — | — | — | — | — | — | — | — | — | — | — | 13,71 | — | — | 9,60 | — |
| Base pour carburéacteur | — | — | — | — | — | — | — | — | — | — | — | — | — | — | — | — | — | — | 13,71 | — | — | — | 4,11 |
| TOTAL | 33,84 | 100 | 100 | 11,76 | 1,09 | 10,67 | 2,97 | 85,27 | 10,72 | 95,77 | 2,52 | 8,20 | 22,07 | 71,40 | 0,27 | 71,17 | 0,04 | 71,13 | 71,13 | 55,49 | 66,16 | 11,53 | 4,11 |

≤Entrées: (1) + (23) = 42,04          ≤Sorties: (12) + (16) + (26) + (29) + (41) + (42) = 42,04

EP 0 237 372 B1

TABLEAU II

| Numéros des conduites de la figure | 1 | 6 | 9 | 11 | 12 | 13 | 16 | 17 | 19 | 20 | 22 | 23 | 26 | 27 | 29 | 30 | 32 | 33 | 35 | 37 | 38 | 41 | 42 |
|---|---|---|---|---|---|---|---|---|---|---|---|---|---|---|---|---|---|---|---|---|---|---|---|
| Hydrogène | — | 9,40 | 10,40 | 10,40 | 1 | 9,40 | — | — | — | — | — | — | — | — | — |  | — | — | — | — | 9,40 | — | — |
| Méthane | — | 0,79 | 0,87 | 0,87 | 0,08 | 0,79 | — | — | — | — | — | — | — | — | — |  | — | — | — | — | 0,79 | — | — |
| Ethane | — | 0,49 | 0,58 | 0,54 | 0,05 | 0,49 | 0,04 | — | — | — |  |  |  |  |  |  |  | — | — | — | 0,49 | — | — |
| Propane | — | 0,08 | 1,44 | 0,09 | 0,01 | 0,08 | 1,35 | — | — | — | — | — | — | — | — |  | — | — | — | — | 0,08 | — | — |
| Propène | — | — | 1,02 | — | — | — | 1,02 | — | — | — | — | — | — | — | — |  | — | — | — | — | — | — | — |
| Isobutane | 11,96 | 48,07 | 37,02 | — | — | — | 1,03 | 35,99 | — | 35,99 | — | — | — | 35,99 | — | 35,99 | — | 35,99 | 36,11 | 36,11 | 36,11 | — | — |
| n-butane | 3,92 | 21,64 | 18,73 | — | — | — | — | 18,73 | — | 18,73 | — | — | — | 18,73 | — | 18,73 | — | 18,73 | 18,73 | 17,72 | 17,72 | 1,01 | — |
| Butène-1 | 4,46 | 4,52 | 4,52 | — | — | — | — | 4,52 | — | 4,52 | — | — | — | 4,52 | — | 4,52 |  | 4,52 | 0,08 | 0,06 | 0,06 | 0,02 | — |
| Isobutène | 5,28 | 5,28 | 16,96 | — | — | — | — | 16,96 | — | 0,10 | — | — | — | 0,10 | — | 0,10 | — | 0,10 | — | — | — | — | — |
| ≤ butènes-2 | 9,73 | 9,73 | 8,40 | — | — | — | — | 8,40 | — | 8,40 | — | — | — | 8,40 | — | 8,40 | — | 8,40 | 0,69 | — | — | 0,69 | — |
| Butadiène-1,3 | — | — | 0,06 | — | — | — | — | 0,06 | — | 0,06 | — | — | — | 0,06 | — | 0,06 | — | 0,06 | — | — | — | — | — |
| Méthanol | — | — | — | — | — | — | — | — | 12,52 | 2,89 | 2,75 | 9,77 | — | 0,14 | 0,14 | — | — | — | — | — | — | — | — |
| MTBE | — | — | — | — | — | — | — | — | 0,26 | 26,75 | 0,26 | — | 26,31 | 0,18 | 0,18 | — | — | — | — | — | — | — | — |
| Eau | — | — | — | — | — | — | — | — | — | — | — | — | — | — | — | 0,04 | 0,04 | — | — | — | — | — | — |
| Supercarburant | — | — | — | — | — | — | — | — | — | — | — | — | — | — | — | — | — | — | 12,19 | — | — | 8,53 | — |
| Base pour carburéacteur | — | — | — | — | — | — | — | — | — | — | — | — | — | — | — | — | — | — | — | — | — | — | 3,66 |
| TOTAL | 35,35 | 100 | 100 | 11,90 | 1,14 | 10,76 | 3,44 | 84,66 | 12,74 | 97,44 | 3,01 | 9,77 | 26,31 | 68,12 | 0,32 | 67,84 | 0,04 | 67,80 | 67,80 | 53,89 | 64,65 | 10,25 | 3,66 |

≤Entrées: (1) + (23) = 45,12          ≤Sorties: (12) + (16) + (26) + 29 + (41) + (42) = 45,12

# EP 0 237 372 B1

**Revendications**

1. Procédé d'obtention de méthyltertiobutylether, d'une essence supercarburant et d'un combustible pour carburéacteur à partir d'une coupe d'hydrocarbures paraffiniques essentiellement à 4 atomes de carbone par molécule, le procédé étant caractérisé en ce que:

a) la dite coupe ou charge fraiche, constituée en majeure partie d'une coupe $C_4$ paraffinique, préalablement séchée, désulfurée et désazotée et qui à ce stade renferme généralement en majeure partie de l'isobutane et du n-butane est envoyée dans une zone de déshydrogénation (7) en phase vapeur de façon à transformer une partie au moins de l'isobutane et du n-butane en isobutène, butène-1 et butènes-2 et butadiène-1,3,

b) l'effluent de la zone de déshydrogénation, refroidi par échange thermique avec la dite charge fraiche, est envoyé dans une première zone de fractionnement (10) en tête de laquelle on recueille un mélange gazeux riche en hydrogène et renfermant du méthane et de l'éthane, ce mélange gazeux étant en majeure partie recyclé vers la zone de déshydrogénation (7), et en fond de laquelle on recueille (conduite (14)) un premier produit que l'on envoie dans une deuxième zone de fractionnement (15) en tête de laquelle on recueille un mélange renfermant notamment du propane et en fond de laquelle on recueille un second produit,

c) le dit second produit, est traité en présence de méthanol en excès dans une zone d'éthérification (18) de façon à transformer la majeure partie de l'isobutène en méthyltertiobutyléther,

d) l'effluent de la zone d'éthérification est soumis à au moins deux étapes de fractionnement dans au moins deux zones successives de fractionnement (21) et (25), en recueillant en fond de la première zone de fractionnement (21) traversée par le dit effluent de la zone d'étherification, du méthanol en excès que l'on recycle en majeure partie vers la zone d'étherification (18) en recueillant en fond de la dernière zone de fractionnement (25) des produits de l'étherification du méthyltertiobutyléther sensiblement pur et en recueillant en tête de la dernière zone de fractionnement (25) des produits de l'étherification un mélange renfermant de l'isobutane, du n-butane, du butène-1, des butènes-2, du butadiène-1,3 et des traces de méthanol et de méthyltertiobutyléther, ce dernier mélange étant envoyé dans une unité (28) de lavage à l'eau acidifiée, afin d'éliminer du méthanol et du méthyltertiobutyléther, une coupe $C_4$ saturée d'eau étant ensuite soutirée de l'unité de lavage à l'eau et envoyée dans une unité de séchage 31, pour éliminer sensiblement la totalité de l'eau de cette dernière coupe $C_4$,

e) on envoie la dernière coupe $C_4$ obtenue à l'étape (d) dans une zone d'oligomérisation (34) en vue de transformer en majeure partie au moins les n-butènes, butènes-2 et butadiène-1,3 en supercarburant et en combustible pour carburéacteur,

f) on envoie l'effluent d'oligomérisation dans au moins deux zones successives de fractionnement et on recueille et tête de la première zone de fractionnement (36) traversée par l'effluent d'oligomérisation un mélange renfermant essentiellement de l'isobutane et du n-butane, ce mélange étant en majeure partie au moins recyclé vers la zone de déshydrogenation, et on recueille en fond de la dernière zone de fractionnement (40) traversée par les produits d'oligomérisation, une coupe riche en combustible pour carburéacteur et en tête de cette dernière zone de fractionnement (40) une coupe riche en essence supercarburant.

2. Procédé selon la revendication 1 dans lequel la charge provient d'une unité de reformage catalytique.

3. Procédé selon la revendication 1 dans lequel la charge provient d'une unité de craquage catalytique.

4. Procédé selon l'une des revendications 1 à 3 dans lequel, dans l'étape (f), la totalité du mélange renfermant essentiellement de l'isobutane et du n-butane est recyclée vers la zone de déshydrogénation.

5. Procédé selon l'une des revendications 1 à 4 dans lequel le combustible pour carburéacteur obtenu à l'étape (f) est soumis à une hydrogénation partielle ou totale.

6. Procédé selon la revendication 5 dans lequel l'hydrogène nécessaire à l'hydrogénation provient au moins en partie du dit mélange gazeux riche en hydrogène défini à l'étape (b) de la revendication 1.

**Patentansprüche**

1. Verfahren zur Herstellung von Methyltertiärbutylether, eines Superbenzins und eines Verbrennungstreibstoffes für Verbrennungsreaktoren (Motoren) ausgehend von einem paraffinischem Kohlenwasserstoffschnitt im wesentlichen mit vier Kohlenstoffatomen pro Molekül, wobei das Verfahren dadurch gekennzeichnet ist, daß

a) der genannte Schnitt oder frische Charge, darstellen zum größeren Teil einen paraffinischen C-Schnitt, vorher getrocknet, entschwefelt und entadsorbiert, und das in diesem Stadium im allgemeinen zumgrößeren Teil Isobutan und n-Butan umfasst, in eine Dehydrogenierungszone (7) geschickt wird in dampfförmiger Phase zur Transformierung wenigstens eines Teils des Isobutans und n-Butans zu Isobuten, 1-Buten und 2-Buten und 1,3-Butadien,

b) das Effluent der Dehydrogenierungszone, abgekühlt durch thermischen Austausch mit genannter frischer Charge, in eine erste Fraktionierungszone (10) geschickt wird, am Kopf welcher man ein gasförmiges Gemisch gewinnt reich an Wasserstoff und umfassend Methan und Ethan, wobei dieses gasförmige Gemisch zum größeren Teil rezyklisiert wird zur Dehydrogenierungszone (7), und am Boden

9

von welcher man (Leitung 14) ein Herstellungsprodukt gewinnt, das man in eine zweite Fraktionierungszone (15) schickt am Kopf von welcher man ein Gemisch gewinnt, umfassend insbesondere Propan und am Boden von welcheren man ein zweites Produkt gewinnt,

c) das genannante zweite Produkt behandelt wird in Anwesenheit von Methanol im Überschuß in eine Veretherungszone (18) zur Transformierung des größeren Teils des Isobutens in Methyltertiärbutylether,

d) das Effluent der Verätherungszone wenigstens zwei Fraktionierungsstufen unterworfen wird in wenigstens zwei aufeinander folgenden Fraktionierungszonen (21 und 25), unter Gewinnung am Boden der ersten Fraktionierungszone (21), die durch das genannte Effluent der Veretherungszone durchströmt wird, überschüssiges Methanol, das man zum größeren Teil zur Veretherungszone (18) rezyklisiert, unter Gewinnung am Boden der letzteren Zone der Fraktionierung (25) von Veretherunsprodukt Methyltertiärbutylether im wesentlichen rein, und indem man am Kopf der letzten Fraktionierungszone (25) der Veretherungsprodukte ein Gemisch gewinnt, umfassend Isobutan, n-Butan, 1-Buten, 2-Buten, 1,3-Butadien und Spuren von Methanol und von Methyltertiärbutylether, wobei dieses letzte Gemisch geschickt wird in eine Wascheinheit (28) mit angesäuertem Wasser, zur Entfernung von Methanol und von Methyltertiärbutylether, wobei ein wassergesättigter C-Schnitt dann abgezogen wird von einer Wasserwascheinheit und in eine Trocknungseinheit (31) geschickt wird zur Entfernung von im wesentlichen der Gesamtheit des Wassers von diesem letzten $C_4$-Schnitt,

e) man diesen letzten $C_4$-Schnitt, erhalten in Stufe d) in eine Oligomerisierungszone (34) schickt zur Transformierung zum größeren Teil wenigstens der n-Butene, 2-Butene und 1,3-Butadien zu Supertreibstoff und zu Verbrennungstreibstoff für Verbrennungsreaktor,

f) man das Effluent der Oligomerisation in wenigstens zwei aufeinander folgende Fraktionierungszonen schickt und man am Kopf der ersten Fraktionierungszone (36), durchquert vom Oligomerisierungseffluent, ein Gemisch gewinnt, umfassend im wesentlichen Isobutan und n-Butan, wobei dieses Gemisch zum größeren Teil wenigstens rezyklisiert wird zur Dehydrogenierungszone, und man am Boden der letzten Fraktionierungszone (40), durchquert von den Oligomerisierungsprodukten, einen Schnitt gewinnt, reich an Brennstoff für Verbrennungsreaktor, und am Kopf dieser letzten Fraktionierungszone (40) einen Schnitt reich an Superbenzintreibstoff.

2. Verfahren gemäß Anspruch 1, in welchem die Charge aus einer katalytischen Reformierungsanlage kommt.

3. Verfahren gemäß Anspruch 1, in welchem die Charge von einer katalytischen Spaltungseinheit kommt.

4. Verfahren gemäß einem der Ansprüche 1 bis 3, in welchem, in Stufe f) die Gesamtheit des Gemisches im wesentlichen Isobutan und n-Butan umfassend, rezyklisiert wird zur Hydrogenierungszone.

5. Verfahren gemäß einem der Ansprüche 1 bis 4, in welchem der Treibstoff für Verbrennungsreaktor, erhalten in Stufe f), einer teilweisen oder vollständigen Hydrogenierung unterworfen wird.

6. Verfahren gemäß Anspruch 5, in welchem der notwendige Wasserstoff zur Hydrierung wenigstens zum Teil von dem genannten gasförmigen Gemisch reich an Wasserstoff, definiert in Stufe b) des Anspruchs 1, kommt.

## Claims

1. A process for obtaining methyltertiobutylether, a high-grade petrol and a jet fuel from a cut of paraffinic hydrocarbons essentially having 4 carbon atoms per molecule, the process being characterised in that:

a) said cut or fresh charge which for the major part comprises a paraffinic $C_4$ cut which has been previously dried, desulphurised and denitrified and which at that stage generally contains for the major part isobutane and n-butane is passed into a vapour phase dehydrogenation zone (7) so as to transform a part at least of the isobutane and n-butane into isobutene, but-1-ene and but-2-enes and buta-1,3-diene,

b) the effluent from the dehydrogenation zone which is cooled by heat exchange with said fresh charge is passed into a first fractionating zone (10), at the head of which is collected a gaseous hydrogen-rich mixture containing methane and ethane, said gaseous mixture being recycled for the major part to the dehydrogenation zone (7), and at the bottom of which is collected (conduit 14)) a first product which is passed into a second fractionating zone (15) at the head of which is collected a mixture containing in particular propane and at the bottom of which a second product is collected,

c) said second product is treated in the presence of methanol in excess in an etherification zone (18) so as to transform the major part of the isobutene into methyltertiobutylether,

d) the effluent from the etherification zone is subjected to at least two fractionating steps in at least two successive fractionating zones (21 and 22), with collection at the bottom of the first fractionating zone (21) through which said effluent from the etherification zone passes, of the excess methanol which is recycled for the major part thereof to the etherification zone (18), with collection at the bottom of the last zone (25) for fractionating the products of the etherification operation of substantially pure methyltertiobutylether and with collection at the head of the last zone (25) for fractionating the products of the etherification operation of a mixture containing isobutane, n-butane, but-1-ene, but-2-enes, buta-1,3-diene and traces of methanol and methyltertiobutylether, the last-mentioned mixture being passed into a unit (28) for washing with acidified water in order to remove methanol and methyltertiobutylether, a water-saturated $C_4$ cut then

being taken from the water washing unit and passed into a drying unit (31) to eliminate substantially the whole of the water from said last C$_4$ cut,

e) the last C$_4$ cut obtained in step d) is passed into an oligomerisation zone (34) for transforming at least the major part of the n-butenes, but-2-enes and buta-1,3-diene into high-grade petrol and jet fuel, and

f) the oligomerisation effluent is passed into at least two successive fractionating zones and a mixture essentially containing isobutane and n-butane is collected at the head of the first fractionating zone (36) through which the oligomerisation effluent passes, the major part at least of said mixture being recycled to the dehydrogenation zone, and a jet fuel-rich cut is collected at the bottom of the last fractionating zone (40) through which the oligomerisation products pass and a high-grade petrol-rich cut is collected at the head of said last fractionating zone (40).

2. A process according to claim 1 wherein the charge comes from a catalytic reforming unit.

3. A process according to claim 1 wherein the charge comes from a catalytic cracking unit.

4. A process according to one of claims 1 to 3 wherein, in step f), the whole of the mixture essentially containing isobutane and n-butane is recycled to the dehydrogenation zone.

5. A process according to one of claims 1 to 4 wherein the jet fuel obtained in step f) is subjected to partial or total hydrogenation.

6. A process according to claim 5, wherein the hydrogen required for the hydrogenation operation comes at least in part from said hydrogen-rich gaseous mixture defined in step (b) of claim 1.

11

PL_unique